# EUROPEAN PATENT SPECIFICATION

(11) **EP 1 869 157 B1**
(45) Date of publication and mention of the grant of the patent: **30.05.2012**
(21) Application number: 06749134.0
(22) Date of filing: 04.04.2006
(51) Int. Cl.: C12M 1/00, C12Q 1/06, C12Q 1/10

(54) **Flexible culture medium bag containing nutrient concentrate**
Flexibler Kulturmedium-Beutel mit Nährstoffkonzentrat
Sac souple pour milieu de culture, contenant un concentré nutritif

(30) Priority: 04.04.2005 US 668020 P
(43) Date of publication of application: 26.12.2007
(73) Proprietor: E.I. DU PONT DE NEMOURS AND COMPANY, Wilmington, DE 19898 (US)
(72) Inventor: STEICHEN, John, Carl, Landenberg, PA 19350 (US); ANDALORO, Bridget, W., Hockessin, DE 19707 (US); KANE, James, P., Jr., Wilmington, DE 19805 (US); VISIOLI, Donna, Lynn, Lower Gwynedd, PA 19002 (US); WANG, Siqun, Wilmington, DE 19803 (US)
(74) Representative: Matthews, Derek Peter
(86) International application number: PCT/US2006/012240
(87) International publication number: WO 2006/107843

(56) References cited:
- EP-A- 0 471 947
- WO-A-98/07828
- US-A- 4 250 256
- US-A- 5 728 542
- US-A1- 2002 168 759
- US-B1- 6 312 930

## Description

This invention relates to a culture medium bag comprising a main compartment and a locus of containment that contains a nutrient concentrate until it is released at the time of use.

### BACKGROUND OF THE INVENTION

Microorganisms can exist in food and in the environment at such low concentrations that they are difficult to measure but still pose a significant health risk. Microbiologists incubate samples in liquid culture media to detect and perform tests for pathogenic microorganisms such as those included in the genesis of *Salmonella, Listeria, Staphylococcus, Clostridium, Campylobacter,* and *Escherichia.* Similar kinds of tests are conducted to detect the presence of microorganisms in samples normally expected to be sterile, such as blood, spinal fluid, medical devices, and a wide variety of industrial materials.

In order to increase the microorganism concentration to measurable levels, a sample for analysis is mixed with a nutrient medium that enables growth of the organism population. This growth may be performed in two stages: (1) homogenization of the nutrient and sample so that they are intimately mixed, and (2) a longer period when the sample and nutrient are exposed to temperatures that foster growth of the target organism. During this second stage additional additives may be introduced into the nutrient medium to create a growth environment unfavorable to non-target organisms. These two stages are referred to as "sample enrichment" in the food industry.

US Patent 6,312,930 discloses a method for detection of specific target bacteria in a complex sample mixture, such as a food sample, by culturing the sample followed by isolation and detection of target bacteria DNA. The target DNA is amplified via PCR amplification protocols and detection is accomplished by gel electrophoresis or by fluorescent means.

Historically, sample enrichment had been conducted in rigid closed containers such as bottles, but a now-preferred approach is to perform the enrichment in flexible plastic bags, which are often referred to as "homogenizer bags" or "Stomacher^{®} bags". These bags offer advantage over solid wall containers in that a machine can mechanically manipulate the bag and its contents thereby performing homogenization of the sample. A typical homogenizer machine has reciprocating paddles that pulverize and mix the sample with the culture medium.

In practice, a microbiologist or technician at a testing laboratory prepares a number of different enrichment media such as sterile liquid culture solutions and buffered diluent solutions with different compositions for different target microorganisms. Media preparation and sterilization is well known to one skilled in the art. Large batches of the media and additives can be prepared and smaller portions are transferred into homogenizer bags for individual analyses. The preparation of culture media at each testing laboratory is expensive, labor intensive, and subject to error, especially during the measuring and transfer operations. This process of media preparation, sterilization, storage, and introduction into the homogenizer bags is considered burdensome by the food and environmental testing industry.

Therefore, prefilled and presterilized rigid containers have been produced. This leaves the laboratory with the task of merely introducing test samples into the containers after they arrive. See, e.g., US Patent 6,379,949.

However, shipment of liquid culture solutions, even in flexible bags, can be undesirable due to the weight and volume of the solutions. Also, a wide variety of analyses needed in the food and environmental testing industry may require many different culture media in terms of dilution, volume, nutrient profile and/or other factors. The wide variety of culture media may result in an overly complex inventory of prefilled bags for manufacture and distribution. Thus, it may be desirable to provide homogenizer bags with measured nutrient concentrates to which purified water can be added at the point of use in the testing laboratory. Alternatively it may be desirable to provide prefilled homogenizer bags wherein the nutrient concentrate and water are contained in separate compartments until the time of use of the bag. Also, providing bags wherein the nutrients are contained in sterile packaging and not in a "broth" until time of use minimizes the chances of the growth of microorganisms that enter the bag adventitiously prior to its intended use. Furthermore, in some cases it may be undesirable to store various nutrient components in solution together, requiring that they be mixed at time of use. For those cases in which the components of the nutrient medium are not compatible with each other for long periods of time, multiple compartments, each containing a component of the final nutrient medium, are desirable.

Flexible pouches with frangible seals have been disclosed in, for example, US Patent 4,602,910, US Patent 5,728,542, US Patent Application 2004/118710, and PCT Patent Application WO91/07503.

### SUMMARY OF THE INVENTION

The invention includes a bag comprising
(a) a first sheet of polymeric film;
(b) a second sheet of polymeric film; wherein said second sheet is superimposed on said first sheet; and wherein said first sheet and said second sheet are sealed to each other directly, or indirectly through an intervening polymeric film, thereby defining a sealed perimeter forming a bag;
(c) one locus of containment within the bag, said locus comprising a nutrient concentrate; and
(d) optionally one or more additional loci of containment within the bag, each of the additional loci comprising an additive selected from the group consisting of indicator compound, dye, quencher, fixative, reagent for extraction or detection of microorganisms, and combinations of two or more thereof; wherein the reagent for extraction or detection of microorganisms is pure or comprises one or more other additives, diluents, phages, components derived from phages, antibodies, poly-histamines, maltose-binding domains, affinity peptides, aptomers, biotins, streptoavidins, or other affinity molecules;
wherein said locus of containment or, when present, at least one of said additional loci of containment comprises (1) at least one frangible seal internal to the perimeter, dividing said bag into separated compartments, or (2) a sachet; wherein said frangible seal or sachet is itself at least partially constructed of a water-reactive polymeric material; and wherein said water-reactive polymeric material dissolves, ruptures, disperses, and/or disintegrates upon contact with water.

This invention also includes a process that can be used for determining the presence of a specific target bacterium suspected of being in a sample using a container as disclosed above. The process comprises
inserting a microorganism-containing sample in a bag as characterized above;
releasing a nutrient concentrate from the locus to produce a culture medium;
incubating the sample in the culture medium to form an enriched complex sample mixture; and
detecting the microorganism in the sample mixture.

### DETAILED DESCRIPTION THE INVENTION

In one embodiment, the locus of containment within the bag comprises a sachet of flexible film in which the nutrient concentrate is contained, wherein at least a portion of the sachet comprises a water- reactive polymeric material.

In alternative embodiments, the locus of containment within the bag comprises a powder, granule, pellet, sheet, plaque, or the like comprising a matrix of water-reactive polymeric material in which the nutrient concentrate is mixed. The locus of containment may comprise a coating of water-reactive polymeric material applied to the nutrient concentrate. The nutrient concentrate may be in powder or granulated form prior to coating with the water-reactive polymeric material. Alternatively, the nutrient medium is formed into a pellet, sheet, plaque or the like prior to the application of the coating of water-reactive polymeric material. As another alternative, the locus of containment is a separate compartment in the pouch defined by a seal formed of a water-reactive material.

In these embodiments, purified and/or sterilized water is added to the main compartment of the homogenizer bag at the time of use, causing the water-reactive polymeric material to be dissolved, ruptured, dispersed and/or disintegrated, releasing the nutrient concentrate and allowing it to commingle with the added water.

In yet another embodiment, the locus of containment comprises a tablet or capsule that can be pulverized and/or dissolved during the homogenizing process. The loci may comprise one or more tablet or capsule that can be pulverized and/or dissolved in a sequential manner for timed releases.

Preferably, the culture medium bag also includes a resealable closure. Optionally the culture medium bag comprises a gusseted base permitting the bag to stand upright when filled.

A container disclosed here can be a "culture medium bag" or "homogenizer bag" used interchangeably herein to refer to a flexible container or bag that can be used to incubate a sample in liquid culture media to foster growth of microorganisms.

A homogenizer machine is a device for blending samples with nutrient media in flexible bags to provide for the culturing of microorganisms. A homogenizer machine comprises a set of paddles that provide a kneading action from outside the bag to blend the sample. See, e.g., US Patent 6,439,759. Homogenizer machines can be available commercially from Seward Ltd., under the Stomacher^{®} tradename.

This invention provides homogenizer bags with containment loci that can be intentionally ruptured so that the contents (e.g. a nutrient concentrate) are released at the time of use. It is possible to locate the containment locus inside a homogenizer bag so that its contents are released when the bag is placed inside a homogenizer machine. Since a sterile homogenizer bag can be provided, the laboratory technician only needs to add the sample and sterile water, place the bag with its contents in the homogenizer, and then retrieve later the homogenized sample.

Homogenizer bags can be made in several sizes for different sample types and sampling procedures, but they are all manipulated in the same manner to achieve homogenization, i.e., the bag is mechanically compressed first in one region and then another. When one region is compressed, the other is released. This process causes the sample and nutrient medium to flow back and forth in the bag. There are various mechanical designs that form flow regimes in the bag, but they all utilize the alternating compression process. Because of the potential release of gas from aerobic microorganisms in the bag, often the bag is not sealed during at least part of the homogenization process, and its contents are exposed to air.

The locus of containment (e.g. a separated compartment or sachet) containing the nutrient concentrate can be placed so that the first compression of the homogenizer paddle ruptures the locus of containment and releases the nutrient into the water. For those cases in which the components of the nutrient medium are not compatible with each other for long periods of time, multiple loci of containment, each containing a component of the final nutrient medium, are desirably located in the homogenizer compression zones. The released nutrient concentrate(s) can dissolve and/or disperse in water to constitute the nutrient medium.

Enrichment of samples for certain microorganisms include an initial phase of growth for all microorganisms followed by a phase of generally suppressed growth for all except the target organism. For example, this second phase might involve the introduction of an additive, such as an antibiotic, that suppresses the growth of non-target organisms, the additive is released from its locus of containment in a step subsequent to the release of the nutrient concentrate.

Other additives that may be released in a step subsequent to the release of the nutrient concentrate may include for example, indicator compounds, dyes, quenchers, fixatives and the like. Other second step additives may be reagents for the extraction and/or detection of microorganisms. Such reagents can be pure, formulated with additives, diluents or in combination with phages (submicroscopic, usually viral organisms that destroy bacteria), components derived from phages, antibodies, poly-histamines or maltose-binding domains or any other affinity peptides, aptomers, biotins or streptoavidins or any other affinity molecules. These formulated reagents can be in the form of liquid, gel, paste, dry powder, granule, or other free-flowable forms. Alternatively, these reagents can be immobilized on solid supports such as particles, particularly magnetic or paramagnetic particles of micron to nanometer sizes.

The invention also contemplates a homogenizer bag with at least one additional locus of containment, located above the compression zones, for the additive(s) as described above to be released in at least one step subsequent to release of the nutrient concentrate. Thus, the additive for the second step can be released by opening its locus of containment at a time after the nutrient concentrate is released. For example, the additive for the second step can be released due to the rupture of a frangible seal in a compartment or sachet either by manual compression or by automatic compression. Other loci of containment for the second step additives, such as compartments with peelable covers, water-soluble sachets, or tablets or capsules are also contemplated in this invention.

The invention can work well in situations where the sample is not easily dissolved in the culture medium. In some cases, in order to obtain a good distribution of the sample, it may need to physically stir or beat the mixture. When the disclosed process is used, the solution of medium and sample can be quickly pulverized or kneaded through the walls of the flexible bag, as needed, without transferring bag contents from one container to another. This reduces the risk of introducing unwanted contaminants. Container-to-container transfer can be a drawback in this situation if rigid containers are used. Additionally, use of a clear bag allows visual inspection of the culture medium and sample at any time during the culturing or test process. Furthermore, use of the invention container can reduce laboratory costs by using prefilled and presterilized culture media without the need of recycling back to the supplier for reuse thereby reducing the incremental cost of rigid container and reducing total mass and volume of waste material.

The nutrient concentrate comprises various nutrients suitable for supporting the growth of microorganisms (e.g., proteins, amino acids, vitamins, sugars, oxygen and the like). It may also comprise such components as inorganic salts, buffers, indicators and the like to facilitate sample culturing and analysis. Preparations of nutrients are available in concentrated form derived from, for example, soybean casein, thioglycolate and brain and/or heart infusions. The nutrient concentrate is typically a solid, and may be in flowable forms such as dusts, powders, granules and the like. Alternatively the concentrate may be compressed into pellets, sheets, plaques or the like, with or without additional binders. Semisolid, paste, gel or concentrated liquid forms may also be suitable for use in some embodiments of this invention.

The bag is preferably made of a material that is resistant to puncturing, suitably transparent to allow visual inspection of the contents, and has a long shelf life. For example, the bag can comprise two sheets of thin film such-that the bag is a "two-sided" bag and may lay flat, one sheet on top of the other when the bag is not filled.

The sheets of polymeric film employed to make the sidewalls of the flexible culture medium bag or the sachet to be employed as a locus of containment within the bag can be either a single layer or multilayer polymeric film. The sheets of film involved in the construction of the bag can be different structure (e.g., one layer can be clear and the other can be opaque). Any such film grade polymeric resin or material as generally known in the art of packaging can be employed. A multilayer polymeric sheet may involve at least three categorical layers, including but not limited to, an outermost structural or abuse layer, an inner barrier layer, and an innermost layer and optionally one or more adhesive or tie layers there between. The innermost layer making contact with and compatible with the intended contents of the bag or sachet is preferably capable of forming both the lock up perimeter seals (i.e., seal strengths greater than 1,500 g/inch, 60gmm) and, for some embodiments of this invention, any frangible seal(s). The innermost layer can be heat-sealable.

The outermost structural or abuse layer can be polyethylene, oriented polyester, or oriented polypropylene, but can also include oriented nylon. This layer preferably is reverse printable and advantageously unaffected by the sealing temperatures used to make the bag and compartments, since the bag is sealed through the entire thickness of the multilayer structure. The thickness of this layer can be selected to control the stiffness of the bag, and may range from about 10 to about 60 µm, preferably about 50 µm.

The inner layer can include one or more barrier layers, depending on which atmospheric conditions (oxygen, humidity, light, and the like) that potentially can affect the product inside the bag. Barrier layers can be metallized oriented polypropylene (PP), aluminum foil, oriented polyethylene terephthalate (PET), ethylene vinyl alcohol (EVOH), nylon or biaxial oriented nylon, blends or composites of the same as well as related copolymers thereof. Barrier layer thickness will depend on the sensitivity of the product and the desired shelf life.

The innermost layer of the package is the sealant. The sealant is selected to have minimum effect on efficacy of the contents, to be unaffected by the product, and to withstand sealing conditions (such as liquid droplets, grease, dust, or the like). The sealant can be a resin that can be bonded to itself (sealed) at temperatures substantially below the melting temperature of the outermost layer so that the outermost layer's appearance may not be affected by the sealing process and may not stick to the jaws of the sealing bar. Sealants used in bags or sachets can include ethylene copolymers, such as low density polyethylene (LDPE), linear low density polyethylene (LLDPE), metallocene polyethylene, or copolymers of ethylene with vinyl acetate or methyl acrylate or copolymers of ethylene and acrylic acid (EAA) or methacrylic acid (EMAA), optionally ionomerized (i.e., partially neutralized with metal ions such as Na, Zn, Mg, or Li). Sealants can also include polypropylene copolymers. Sealant layers can be 25 to 100 µm thick. For some embodiments of the invention, the sealant preferably forms a frangible seal that ruptures and bursts by compression of the bag.

During the manufacture of the polymeric film sheet to be used in making the bag, co-extrudable adhesives are optionally used between functional layers to adhere the layers to each other and to provide structural integrity. These include, but are not limited to, polymers and copolymers of ethylene or propylene modified with or grafted with unsaturated carboxylic acid groups such a maleic anhydride or maleic acid and the like. Also, to provide additional thickness (if desired by the consumer for a particular application), bulk layers of polyolefin or chopped remnants of the multilayer film trimmed during bag fabrication can be incorporated within the multilayer structure.

In some cases, the functions of structural, barrier and/or sealant layers may be combined in a single polymeric layer. Of note are culture medium bags of this invention wherein the films used in the bags comprise a single layer of polyethylene. Also of note are bags wherein the films used in the bags comprise an outer layer of nylon and an inner layer of ethylene/vinyl acetate copolymer (EVA). Other suitable multilayer materials for bag construction include (from outermost to innermost layer):

### Water-Reactive Materials

The locus of containment comprises a water-reactive material. Water-reactive material means a material that dissolves, ruptures, disperses and/or disintegrates upon contact with water, so as to allow the nutrient concentrate contained therein to be released into the water and form a liquid culture medium. Preferably, the material is water-soluble, such as a water-soluble polymeric material.

Sachets are preferably made from a water-soluble film. The sachet may be made from two overlaid sheets of water-soluble film that are sealed together. Alternatively, the sachet may comprise a sheet of water-soluble film sealed to a sheet of film that is not water-reactive. This alternative may be useful in preparing a sachet with an extended tab for sealing between the sheets that form the homogenizer bag, wherein the extended tab comprises the film that is not water-reactive. The sachet may have a soluble seal that dissolves to release the bag contents.

The water-soluble film useful for these embodiments has a solubility in water of at least 50%, at least 75%, or even at least 95%. Solubility can be determined as follows. Fifty grams ± 0.1 g of material is added to a 400-ml beaker of known weight, and 245 ml ± 1 ml of distilled water is added. This is stirred vigorously on magnetic stirrer set at 600 rpm for 30 minutes. Then, the mixture is filtered through a folded qualitative sintered-glass filter with the known pore sizes (typically less than 50 µm) to remove the insoluble material. The water is dried off from the collected filtrate by any conventional method, and the weight of the polymer residue is determined (which is the dissolved or dispersed fraction). Then, the % solubility or dispersability can be calculated.

Preferred materials are films of polymeric materials, e.g. polymers that are formed into a film or sheet. The film can, for example, be obtained by casting, blow-molding, extrusion or blow extrusion of the polymer material, as known in the art. Preferred polymers, copolymers or derivatives thereof are selected from polyvinyl alcohols, polyvinyl pyrrolidone, polyalkylene oxides, polyacrylamide, polyacrylic acid, cellulose, cellulose ethers, cellulose esters, cellulose amides, polyvinyl acetates, polycarboxylic acids and salts, polyaminoacids or peptides, polyamides, polyacrylamide, copolymers of maleic/acrylic acids, polysaccharides including starch and gelatine, natural gums such as xanthum and carragum. The polymer can be polyacrylates and water-soluble acrylate copolymers, methylcellulose, carboxymethylcellulose sodium, dextrin, ethylcellulose, hydroxyethyl cellulose, hydroxypropyl methylcellulose, maltodextrin, polymethacrylates, even more preferably polyvinyl alcohols, polyvinyl alcohol copolymers and hydroxypropyl methyl cellulose (HPMC). The polymer can have any weight average molecular weight, preferably from about 1000 to 1,000,000, or even from 10,000 to 300,000 or even from 15,000 to 200,000 or even from 20,000 to 150,000.

Mixtures of polymers can also be used. This may be beneficial to control the mechanical and/or dissolution properties of the containment locus, depending on the application thereof and the required needs. For example, one polymer material has a higher water-solubility than another polymer material, and/or one polymer material has a higher mechanical strength than another polymer material. It may be preferred using a mixture of polymers, having different weight average molecular weights, for example a mixture of polyvinyl alcohol (PVA) or a copolymer thereof of a weight average molecular weight of 10,000-40,000, preferably around 20,000, and of PVA or copolymer thereof, with a weight average molecular weight of about 100,000 to 300,000, preferably around 150,000.

Also useful are polymer blend compositions, for example comprising a hydrolytically degradable and water-soluble polymer blend such as polylactide and polyvinyl alcohol, achieved by the mixing of polylactide and polyvinyl alcohol, typically comprising 1-35% by weight polylactide and approximately from 65% to 99% by weight polyvinyl alcohol, if the material is to be water-soluble.

The polymer can present in the film from 60% to 98%, or 80% to 90%, hydrolyzed, to improve the dissolution of the material, and/or that the levels of plasticizer, including water, in the film are varied such that the dissolution is adjusted as required.

Also preferred is PVA film where the level of polymer in the film can be at least 60%. Such films can comprise a PVA polymer with similar properties to the film known under the trade reference M8630 or CXP4087, as sold by Chris-Craft Industrial Products of Gary, Indiana, US. Examples also include the materials M8630 and/or CXP4087 themselves. Other example PVA films are also available as "Solublon PT30" and "Solublon KA40" from Aicello Chemical Co., Ltd., Aichi, Japan.

Plasticizers can include water glycerol, ethylene glycol, diethyleneglycol, propylene glycol, sorbitol, and mixtures thereof. Other additives can be stabilizers, disintegrating aids, etc.

The sachet can be made of a material which is stretchable, as set out herein. This facilitates the closure of the open sachet, when it is filled over than 90% or even 95% by volume or even 100% or even over-filled. The material is preferably elastic, to ensure tight packing and fixation of the nutrient concentrate therein during handling, e.g., to ensure no (additional) head space can be formed after closure of the sachet. Preferred stretchable materials have a maximum stretching degree of at least 150%, at least 200%, or at least 400% as determined by comparison of the original length of a piece of material just prior to rupture due to stretching, when a force of from about 1 to about 20 Newtons is applied to a piece of film with a width of 1 cm. Preferably, the material is such that it has a stretching degree as before, when a force of from about 2 to about 12 Newtons, or about 3 to about 8 Newtons, is used. For example, a piece of film with a length of 10 cm and a width of 1 cm and a thickness of 40 µm is stretched lengthwise with an increasing stress, up to the point that it ruptures. The extent of elongation just before rupture can be determined by continuously measuring the length and the degree of stretching can be calculated. For example, a piece of film with an original length of 10 cm that is stretched with a force of 9.2 Newton to 52 cm just before breaking, has a maximum stretching degree of 520%.

The force to stretch such a piece of film (10 cm x 1 cm x 40 microns) to a degree of 200% can be within the ranges disclosed above. This can ensure that the elastic force remaining in the film after forming the sachet or closing the sachet is high enough to pack the nutrient concentrate tightly within the sachet (but not so high that the film cannot be drawn into a vacuum mold of reasonable depth, when the sachet is made by a process involving the use of vacuum, such as by vacuum-forming or thermo-forming). The stretchable material is defined by a degree of stretching measured when it is not present as a closed sachet. However, the material can be stretched when forming or closing the sachet. This can for example been seen by printing a grid onto the material, e.g. film, prior to stretching, then forming a sachet; it can be seen that squares of the grid are elongated and thus stretched.

The elasticity of the stretchable material can be defined as the "elasticity recovery". This can be determined by stretching the material for example to an elongation of 200%, as set out above, and measuring the length of the material after release of the stretching force. For example, a piece of film of a length of 10 cm and width 1 cm and thickness of 40 µm is stretched lengthways to 20 cm (200% elongation) with a force of 2.8 Newtons (as above), and then the force is removed. The film snaps back to a length of 12 cm, which indicates an 80% elastic recovery. The sachet material can have an elasticity recovery of from about 20% to about 100%, about 50% to about 100%, about 60% to about 100%, about 75% to about 100%, or about 80% to about 100%.

The degree of stretching can be non-uniform over the sachet, due to the formation and closing process. For example, when a film is positioned in a mold and an open sachet is formed by vacuum forming, the part of the film in the bottom of the mold, furthest removed form the points of closing, may be stretched more than in the top part. A stretching action, when using stretchable, elastic, or both, material stretches the material non-uniformly resulting in a sachet which has a non-uniform thickness. This may allow control of the dissolution/disintegration or dispersion of the sachets in the water added to the culture medium bag. The material can be stretched such that the thickness variation in the sachet formed of the stretched material is from 10 to 1000%, 20% to 600%, 40% to 500%, or 60% to 400%. This can be measured by any method, for example by use of an appropriate micrometer.

Alternative embodiments comprising water-reactive polymers include matrices or coatings of water-reactive material that enclose or envelop the nutrient concentrate solids so that they are not exposed to the air and adventitious microorganisms prior to the time of use. Another alternative embodiment comprises a separated compartment of the bag defined by a seal formed from a water-reactive material. The water-reactive materials for these embodiments may comprise the materials already described for the water-soluble films.

Preferably, the water-reactive sachet, matrix, coating or seal begins releasing the nutrient concentrate almost immediately upon contacting water during sample culture preparation. For example, the sachet begins releasing the concentrate from about 1 second to about 120 seconds, or about 5 seconds to about 60 seconds, after contacting the water.

### Bag Assembly

The sheet(s) of polymeric film (i.e., the so-called "web stock") used to prepare the bag of this invention or sachets may be produced using any combinations of the processes generally known in the art, such as monolayer or multilayer casting, blowing film, extrusion lamination, and adhesive lamination and combinations thereof. Processing aids as generally known in the art, including by way of example but not limited thereto; slip agents (such as amide waxes), antiblocking agents (such as silica), and antioxidants (such as hindered phenols), may be incorporated in the web stock if required to facilitate either manufacture of the film or bag formation. Bags are formed from web stock either by cutting and heat-sealing separate pieces of web stock or by a combination of folding and heat-sealing with cutting. Although the invention is defined as comprising a first sheet and a second sheet of polymeric film, a single web of film may be folded onto itself to provide two overlying sheets, or a tube of film may be formed such that two overlying portions of the tube provide the equivalent of two sheets of film. The heat-sealed perimeter of the bag can be achieved by superimposing the first and second sheets of polymeric film and then heat-sealing each directly to the other or heat-sealing them indirectly through the use of an intervening third polymeric film, as generally known and practiced in the art.

Bag or bag-making equipment such as that made by Totani Corporation, Kyoto, Japan or Klockner Barlelt Co., Gordonsville, VA, USA, can be used.

Bags are desirably prepared in a manner to provide a hermetic seal completely around the perimeter to fully enclose the interior of the bag and its contents prior to time of use. A complete perimeter seal may maintain the interior of the bag in a sterile condition. The bag may be cut or torn open below the top perimeter seal at the time of use to introduce the test sample and water needed to constitute the culture medium (if not already provided in the bag). Optionally, the bag comprises a guiding means for facilitating the opening of the bag for adding a culture sample. Such guiding means comprises at least one notch, perforation or a combination thereof incorporated in the bag near the top seal.

A bag can have a resealable closure near the top seal such as the resealable opening in the form of a "zipper" or "ziplock" closure, and other ways of sealing the bag. A "ziplock" closure for the bag allows for convenient opening and resealing of the bag when adding a sample to the bag for culturing.

An alternative method of resealing the bag is to use a closure wire instead of a "ziplock" closure. The closure wire is connected to the upper portion of one of the sheets, and the closure wire has a length that exceeds the width of the bag. To reseal the bag after the sample has been added, the top edges of the bag, including the opening for sample insertion, are flattened together and then rolled about the closure wire. The ends of the closure wire are then wrapped over the rolled portion to prevent that portion from unrolling.

Alternatively, the bag can be reclosed by rolling its upper edges together and clipping the rolled portion with a spring clamp. An alternative method of sealing the bag after the sample has been added involves simply bonding the upper edges together by heat-sealing or the like to form an airtight seal.

Bags can be partially assembled before introduction of the nutrient concentrate (i.e. as many operations in bag assembly as possible are accomplished prior to introduction of the nutrient concentrate). For example, it may be desirable to assemble "blank" bags in which the top perimeter seal and optional resealable closure are in place before the locus of containment for the nutrient concentrate is introduced into the bag through an opening in the lower or bottom portions of the perimeter seal. Partial assembly can produce nonspecific blank bags that can be customized with different test-specific nutrient concentrate and optional additive packages.

Bags may be prepared in a variety of sizes depending on the test to be performed. An example homogenizer bag can be about 18 cm (7 inches) wide and 29 cm (11.5 inches) high. When a bag is placed in a suitable homogenizer machine, it is held by the cabinet door or clamp at a pressure point about 24 cm (9.5 inches) above the bottom of the bag. The area of the bag affected by the paddles (the compression zone) extends to about 19 cm (7.5 inches) above the bottom of the bag. The area of the bag between the pressure point and the compression zone is generally not subject to compression under normal use.

In some embodiments, the nutrient concentrate is introduced into a separated compartment through an opening in the perimeter seal, as disclosed below, and the bag is then sealed. In alternative embodiments, the locus of containment, such as a sachet, may be prepared in an operation separate from bag formation. In those cases, the locus of containment is inserted into the bag through an opening in the perimeter seal prior to sealing the bag.

One or more frangible compartments can be installed either during or after bag formation. As disclosed above, the frangible compartment can be formed by heat-sealing the overlying sheets at temperatures lower than that required to provide the lock-up perimeter seal. A frangible seal may run between two points on the perimeter seal such that the frangible seal and the portion of the perimeter seal between the points defines a separated compartment. A portion of the perimeter seal is left unsealed to provide an opening to introduce the nutrient concentrates. After the nutrient concentrate is introduced into the separated compartment, the opening in the perimeter seal is sealed to provide a closed compartment. Similarly, a separated compartment can be defined by zone-printing a water-reactive material on the inner surface of one sheet forming the bag and sealing it to the inner surface of the other sheet forming the bag. The frangible compartment can be incorporated in the bag in a region of the bag so that it can be readily compressed by the paddles of homogenizer machines (in the example bag described above, in the area between the bottom of the bag to about 19 cm (7.5 inches) above the bottom, preferably between the bottom of the bag to about 8 cm (3 inches) above the bottom).

Sachets containing nutrient concentrate can be inserted into the bag through an opening in the perimeter seal. The sachets may be loosely held inside the bag, e.g., incorporated into the bag fixed in a region of the bag so that they can be readily compressed by the paddles of homogenizer machines. Sachets can be prepared with at least one extended tab that can be inserted between the first sheet of polymeric film and the second sheet of polymeric film on the perimeter of the bag prior to heat-sealing to fix their position. For example, an extended tab can be sealed between the sheets of webstock in the bottom seal or a side seal. Alternatively, extended tabs on each end of the sachet can be sealed in a portion of each side seal so that the sachet spans the width of the bag. Other ways of fixing the sachet to the bag can include use of adhesives.

In other embodiments, such as for example, sheets, pellets, granules and capsules, the locus of containment may be simply inserted into the bag prior to heat-sealing the opening in the perimeter of the bag. Sheets, pellets and the like may optionally be adhered to a specific location in the interior of the bag, by for example, a hot-melt adhesive.

Multiple sachets or compartments comprising either frangible seals or water-reactive materials, each having a component of the nutrient concentrate, may also be incorporated into a strip of polymeric material. The strip of sachets or compartments can be sealed in a portion of each side seal so that the strip spans the width of the bag in a region of the bag allowing the paddles of homogenizer machines compress them.

As disclosed above, a bag may comprise one or more additional loci of containment for additives. These loci can be located in a region of the bag above the compression zones (in the example bag disclosed above, in the area between about 19 cm above the bottom to about 24 cm from the bottom), so that they can be added in a second step subsequent to the nutrient medium constitution step. These loci can be incorporated into a strip of polymeric material that can be sealed in a portion of each side seal near the upper end of the bag above the compression zones.

The invention also includes a gusseted bag, which is one having a gusseted (pleated) base that allows the bag to stand alone without any external support. The gusseted bag can comprise at least two sheets of packaging film that lay one on top of the other when the bag is not filled. The lower portion or region of the sheets, the same or different, are connected together and closed to form the gusseted base. For example, one sheet may be opaque, optionally with graphic elements, and another sheet may be transparent to allow visualization of the contents of the bag. A particular form of stand-up bag comprises three sheets of packaging film, one of which forms the bottom of the bag and is gusseted, and two that form the sides of the bag. The sheets are joined together by two seams at the bottom of the bag and perimeter seams at the sides. The seams provide sufficient rigidity to the bag to enable it to stand upright. After filling, the constituted liquid culture medium applies outward pressure to opposite sides of the bag, forcing the sides away from each other. At the base of the bag, the bottom or gusset unfolds. The gusset both defines the floor of a liquid holding vessel and constrains the outward movement of sides of the bag. The lower side edges are stiffened by the opening of the gusset and define a stable base that enables the bag to rest on any flat surface in a vertical arrangement. When the bag is closed, it is easy to move from place to place and can be placed on shelves or in boxes along with other bags of the same construction.

An optional mesh bag may be placed inside the culture medium bag. The mesh bag can be made of a mesh-type cloth or similar material that may enable the medium and microorganisms in the medium to pass through its walls, but at the same time, serves as a filter for particulate matter. The mesh bag may filter particles so that if a serological pipette is used to remove bag contents, the pipette does not become clogged. Optionally, a pipette sock may be attached to one of the inner walls of the bag. An upper end of the sock is open for receiving a pipette and the lower end is closed. For example, in use, a pipette may be inserted into the sock and the medium may be drawn from inside the pipette sock as the filtration medium. Any material suitable for use as a filtration medium in the context just disclosed may be used as a pipette sock.

### Bag Sterilization

Sterilization of the bag and nutrient concentrate occurs in a clean room under stringent conditions. The sterilized concentrate can be placed in the bag, either into a separated compartment or contained within a sachet, as disclosed above. A bag can be sterilized by means known to one skilled in the art such as in an autoclave at a temperature of 121°C, a pressure of 15 psi, and 100 % steam. The bag and/or sachets may also be sterilized by irradiation, a conventional procedure that is familiar to a skilled person.

A non-sterile nutrient concentrate can be introduced into a non-sterile bag and then both items are subjected to irradiation treatment as a single unit. Gamma rays or electron radiation may also render the unit sterile. It is preferred that any contaminants not be allowed to grow to significant levels prior to radiation treatment unless the radiation dosage is increased to completely kill these contaminants and may render one or more nutrients in the medium incapable of supporting the growth of desired microorganisms when subsequently used for culturing. Excessive growth of these contaminants prior to sterilization may result in the creation and accumulation of toxic waste products that cannot be removed by sterilization, but may nevertheless restrict or prevent the growth of microorganisms during culturing. Control of the presterilization growth of contaminants can include sterilizing within a short period of time after filling (e.g., 48 hours) or by refrigerating to restrict the growth of the contaminants.

Radiation dosage required for sterilization is well known to one skilled in the art and may depend on bag material and type of culture medium. For example, a gamma radiation dose of 2.5 Mrads may be sufficient to kill contaminants. Gamma radiation in the range of 15 to 30 kGy can be used.

### Testing Samples For Target Organisms

This invention also includes a process for determining a specific target bacterium suspected of being in a sample using the culture medium bag disclosed above. The process can comprises (1) inserting a sample in the bag, (2) releasing a nutrient concentrate from its locus of containment and constituting a culture medium; (3) incubating the sample in the culture medium to form an enriched complex sample mixture; and (4) detecting the presence of the target bacterium in the sample mixture. The complex mixture may comprise a non-selectively enriched food matrix.

After preparation, the culture medium bag is shipped to a laboratory or testing facility for testing a sample (sometimes called a "culture sample"). The culture medium bag is opened and the culture sample is inserted into it along with water as needed to constitute the liquid culture medium from the nutrient concentrate. The bag is sealed and the nutrient concentrate is released from its locus of containment by reaction with the water in the case of water-reactive embodiments and/or by the action of manual compression or compression by the homogenizer machine to provide the liquid culture medium. It is then incubated according to known procedures and sample type. After incubation, the cultured sample is inspected and tested for sample assessment according to procedures well known in the art. For example, detecting target bacteria in the complex sample mixture can comprise (i) obtaining total target bacteria DNA from the target bacteria; (ii) contacting the total target bacteria DNA with a test replication composition to form a first reaction mixture and with a positive control replication composition to form a second reaction mixture; (iii) thermocycling the first and second reaction mixtures thereby producing DNA amplification products consisting of either or both (amplified total target bacteria DNA to produce multiple copies of target DNA or amplified control nucleic acid fragment); and (iv) detecting the amplification products wherein the presence of amplified control nucleic acid fragment alone indicates a successful reaction and wherein the presence of multiple copies of target DNA indicates the presence of the target bacteria in the sample. For example, the presence of amplification products may be detected by fluorescent means, gel electrophoresis, or both.

The test replication composition may comprise (a) a polymerase and (b) a primer pair consisting of a first primer and a second primer, each primer capable of hybridizing to a portion of the total target bacteria DNA; (c) reagents and buffers necessary to effect DNA amplification. The positive control replication composition may comprise (a) a polymerase, (b) at least one control nucleic acid fragment, (c) a single primer capable of hybridizing to a portion of the control nucleic acid fragment, and (d) reagents and buffers necessary to effect DNA amplification. The test replication composition, positive control replication composition, or both may be provided in a tablet. The single primer may be the same as either the first or second primer. The number of the control nucleic acid fragments may be from 1 to 10.

The test replication composition, positive control replication composition, or both, may comprise an intercalating agent such as an asymmetrical cyanine dye. The cyanine dye may be Quinolinium; 1,1'-[1,3-propanediylbis[(dimethyliminio)-3,1-propanediyl]]bis[4-[(3-methy 1-2(3H)-benzotaidhiazolylidene)methyl]]-,tetraiodide, available under the tradename TO-TO-1™; Quinolinium, 4-[(3-methyl-2(3H)-benzoxazolylidene)methyl]-1-[3-(trimethylammonio)propyl] -,diiodide available under the tradename YO-PRO-1™; or combinations of two or more thereof.

The target bacteria may be pathogenic bacteria such as those including genus of *Salmonella, Listeria, Escherichia, Campylobacter, Clostridium, staphylococcus,* or combinations of two or more thereof.

### EXAMPLES

The following Examples are merely illustrative, and are not to be construed as limiting to the scope of the invention.

### Example 1

Coliform testing of water and wastewater samples is accomplished by adding 100 ml of the water sample to a homogenizer bag containing a nutrient concentrate supplemented with the reagent o-nitropbenyl beta-D-galactopyranoside (ONPG) in a water-reactive sachet. The concentrate is made as a powder such that the addition of the water brings the constituents up to the correct final concentrations. After the sachet has ruptured, releasing the nutrient concentrate, the sample in the bag is incubated at 35 °C for 24 hours. If coliform bacteria are present, the colorless ONPG compound is converted to a yellow color.

### Example 2

*E. coli* testing of water and wastewater samples is accomplished by adding 100 ml of the water sample to a bag containing a sheet comprising a nutrient concentrate in a water-reactive matrix. The nutrient concentrate (e.g., lauryl sulfate), supplemented with the reagent 4-methylumbelliferyl-beta-D-glucuronide (MUG), is prepared such that the addition of the water brings the constituents up to the correct final concentrations. After the water-reactive matrix dissolves, releasing the concentrate, the sample in the bag is incubated at 35 °C for 24 hours. If E. *coli* bacteria are present, the colorless MUG compound is converted to a fluorescent bluish compound that is observed under long wave (365 mm) ultraviolet light.

### Example 3

Environmental surface samples such as floors, drains, and equipment in a food company plant, are analyzed for the presence of microorganisms such as *E. coli, Listeria* species, and *Salmonella typhimurium.* These samples are collected using sterile swabs or sponges. Swabs or sponges can be added to a homogenizer bag containing nutrient concentrate granules coated with a water-reactive coating together with 100 ml of sterile water. The nutrient concentrate comprises lauryl sulfate concentrate with MUG (for *E. coli*), UJVM concentrate (for *Listeria*), or Buffered Peptone concentrate (for *Salmonella*)*.* The broth resulting after dissolution of the water-reactive coating and dispersion of the concentrate and the swab or sponge is incubated for 18 to 24 hours at 35 °C. After incubation, the liquid culture medium is observed for the presence of bluish fluorescent material under long wave (354 mm) ultraviolet light for the E. *coli* test, or aliquots of the growth medium are removed and analyzed using a rapid methods procedure such as an enzyme immunoassay, a gene probe detection method, or by a traditional pure culture method as described in manuals such as the BAM for *Listeria* and *Salmonella* testing.

### Example 4

Fifty grams of food sample such as meat is added to a flexible, plastic bag that incorporates a plastic mesh bag and contains a capsule filled with sterile concentrate sufficient to make 450 ml of Butterfield's Phosphate Buffer and 450 ml of water is added. With even distribution of the sample, this will dilute the meat 1:10. The bag is placed into a machine with reciprocating paddles and blended for 1 minute, pulverizing the capsule and releasing the concentrate. A one-ml aliquot is removed from the bag using a serological pipette by accessing the diluent on the opposing side of the mesh bag from the meat. This minimizes the possibility of particulates clogging the serological pipette. A quantitative analysis is performed using a pour or spread plate procedure or using a most probable number (MPN) procedure. Such procedures are well known to those skilled in the art of food analysis.

## Claims

1. A bag comprising
(a) a first sheet of polymeric film;
(b) a second sheet of polymeric film; wherein said second sheet is superimposed on said first sheet; and wherein said first sheet and said second sheet are sealed to each other directly, or indirectly through an intervening polymeric film, thereby defining a sealed perimeter forming a bag;
(c) one locus of containment within the bag, said locus comprising a nutrient concentrate; and
(d) optionally one or more additional loci of containment within the bag, each of the additional loci comprising an additive selected from the group consisting of indicator compound, dye, quencher, fixative, reagent for extraction or detection of microorganisms, and combinations of two or more thereof; wherein the reagent for extraction or detection of microorganisms is pure or comprises one or more other additives, diluents, phages, components derived from phages, antibodies, poly-histamines, maltose-binding domains, affinity peptides, aptomers, biotins, streptoavidins, or other affinity molecules;
wherein said locus of containment or, when present, at least one of said additional loci of containment comprises (1) at least one frangible seal internal to the perimeter, dividing said bag into separated compartments, or (2) a sachet; wherein said frangible seal or sachet is itself at least partially constructed of a water-reactive polymeric material; and wherein said water-reactive polymeric material dissolves, ruptures, disperses, and/or disintegrates upon contact with water.

2. The bag of claim 1 wherein the bag includes a resealable closure.

3. The bag of claim 1 or 2 wherein
the locus comprises a powder, granule, pellet, sheet, or plaque comprising a matrix of water-reactive polymeric material in which the nutrient concentrate is mixed.

4. The bag of claim 3 wherein the nutrient medium is formed into a pellet, sheet, or plaque before the application of the coating of water-reactive polymeric material.

5. The bag of claim 1, 2, 3 or 4 wherein the locus or one of the additional loci comprises a tablet or capsule capable of being pulverized and/or dissolved during a homogenizing process.

6. The bag of claim 1, 2, 3, 4 or 5 comprising the one or more additional loci of containment wherein the one or more additional loci comprises an antibiotic and the reagent is in the form of liquid, gel, paste, dry powder, granule, or other free-flowable form.

7. The bag of claim 6 wherein the reagent is immobilized on a solid support comprising magnetic particles or paramagnetic particles.

8. The bag of claim 1, 2, 3, 4, 5, 6 or 7 comprising (1) a guiding means for facilitating the opening of the bag for adding a culture sample wherein the guiding means comprises at least one notch, perforation or a combination thereof incorporated in the bag near the top seal or (2) a gusseted base permitting the bag to stand upright when filled.

9. A process comprising
inserting a microorganism-containing sample in a bag as **characterized in** claim 1, 2, 3, 4, 5, 6, 7 or 8;
releasing a nutrient concentrate from the locus to produce a culture medium;
incubating the sample in the culture medium to form an enriched complex sample mixture; and
detecting the microorganism in the sample mixture.

10. The process of claim 9 wherein the detecting comprises obtaining total DNA from the microorganism; contacting the total DNA with a test replication composition to form a first reaction mixture and with a positive control replication composition to form a second reaction mixture; thermocycling the first reaction mixture and second reaction mixture thereby producing DNA amplification product; and detecting the amplification product wherein the test replication composition may comprise a polymerase, a primer pair and a reagent; the positive control replication composition comprise a polymerase, at least one control nucleic acid fragment, a single primer capable of hybridizing to a portion of the control nucleic acid fragment, and the reagent; the reagent is necessary to effect DNA amplification; and the microorganism includes the genus of *Campylobacter*, *Listeria, Escherichia, Staphylococcus,* or *Clostridium.*

11. The process of claim 9 or 10 wherein the complex sample mixture comprises a non-selectively enriched food matrix; the test replication composition or positive control replication composition preferably is in a tablet form or comprises an intercalating agent; the presence of the amplification products is detected by fluorescent means; and the intercalating agent is preferably an asymmetrical cyanine dye including Quinolinium, 1,1'-[1,3-propanediylbis[(dimethyliminio)-3,1-propanediyl]]bis[4-[(3-methy l-2(3H)-benzothiazolylidene)methyl]]-,tetraiodide, or Quinolinium, 4-[(3-methyl-2(3H)-benzoxazolylidene)methyl]-1-[3-(trimethylammonio)propyl]-diiodide.

12. The process of claim 10 or 11 wherein the single primer is the same as the first primer or the second primer; and the number of the control nucleic acid fragments is from 1 to 10.

## Patentansprüche

1. Beutel umfassend
(a) ein erstes Blatt Polymerfolie;
(b) ein zweites Blatt Polymerfolie; wobei das zweite Blatt über das erste Blatt gelegt ist; und wobei das erste Blatt und das zweite Blatt direkt miteinander versiegelt sind oder indirekt durch eine dazwischenliegende Polymerfolie, wodurch ein versiegelter Perimeter, der einen Beutel bildet, definiert wird;
(c) einen beinhaltenden Ort innerhalb des Beutels, wobei der Ort ein Nährstoffkonzentrat umfasst; und
(d) wahlweise einen oder mehrere zusätzliche beinhaltende Orte innerhalb des Beutels, wobei jeder der zusätzlichen Orte ein Zusatzmittel umfasst ausgewählt aus der Gruppe bestehend aus einer Indikatorverbindung, einem Farbstoff, Quencher, Fixiermittel, Reagens zur Extraktion oder Erfassung von Mikroorganismen und Kombinationen von zwei oder mehreren davon; wobei das Reagens zur Extraktion oder Erfassung von Mikroorganismen rein ist oder ein oder mehrere andere Zusatzmittel, Verdünnungsmittel, Phagen, von Phagen abgeleitete Komponenten, Antikörper, Polyhistamine, Maltosebindungsdomänen, Affinitätspeptide Aptomere, Biotine, Streptoavidine oder andere Affinitätsmoleküle umfasst;
wobei der beinhaltende Ort oder, liegen sie vor, mindestens einer der zusätzlichen beinhaltenden Orte (1) mindestens eine zerbrechliche Versiegelung innerhalb des Perimeters, die den Beutel in einzelne Kompartimente aufteilt, oder (2) ein Beutelchen umfasst; wobei die zerbrechliche Versiegelung oder das Beutelchen selbst mindestens teilweise aus einem mit Wasser reaktionsfähigen Polymermaterial hergestellt ist; und wobei das mit Wasser reaktionsfähige Polymermaterial sich löst, zerreist, sich dispergiert und/oder zerfällt wenn es mit Wasser in Kontakt kommt.

2. Beutel nach Anspruch 1, wobei der Beutel einen wiederverschließbaren Verschluss umfasst.

3. Beutel nach Anspruch 1 oder 2, wobei
der Ort ein Pulver, Granulat, Kügelchen, Blatt oder Platte umfasst, die eine Matrix von mit Wasser reaktionsfähigem Polymermaterial umfasst, in dem das Nährstoffkonzentrat gemischt ist.

4. Beutel nach Anspruch 3, wobei das Nährstoffmedium zu einem Kügelchen, Blatt oder einer Platte vor Aufbringen der Beschichtung aus mit Wasser reaktionsfähigem Polymermaterial geformt wird.

5. Beutel nach Anspruch 1, 2, 3 oder 4, wobei der Ort oder einer der zusätzlichen Orte eine Tablette oder Kapsel umfasst, die dazu fähig ist, während eines Homogenisierungsvorgangs pulverisiert und/oder gelöst zu werden.

6. Beutel nach Anspruch 1, 2, 3, 4 oder 5, umfassend den einen oder mehrere zusätzliche beinhaltende Orte, wobei der eine oder die mehreren zusätzlichen Orte ein Antibiotikum umfasst/umfassen und das Reagens in Form einer Flüssigkeit, eines Gels, einer Paste, eines Trockenpulvers, Granulats oder in einer anderen freifließenden Form vorliegt.

7. Beutel nach Anspruch 6, wobei das Reagens auf einem festen Träger immobilisiert ist, der magnetische Teilchen oder paramagnetische Teilchen umfasst.

8. Beutel nach Anspruch 1, 2, 3, 4, 5, 6 oder 7 umfassend (1) ein Führungsmittel zum Erleichtern des Öffnens des Beutels zum Hinzugeben einer Kulturprobe, wobei das Führungsmittel mindestens eine Kerbe, Perforation oder eine Kombination davon umfasst, die in den Beutel in der Nähe der oberen Versiegelung eingearbeitet ist, oder (2) einen mit einem Zwickel versehenen Boden, der es dem Beutel gestattet, im gefüllten Zustand aufrecht zu stehen.

9. Verfahren umfassend
das Eingeben einer einen Mikroorganismus enthaltenden Probe in einen Beutel wie in Anspruch 1, 2, 3, 4, 5, 6, 7 oder 8 gekennzeichnet;
das Freisetzen eines Nährstoffkonzentrats aus dem Ort unter Bildung eines Kulturmediums;
das Inkubieren der Probe in dem Kulturmedium unter Bildung einer angereicherten komplexen Probemischung; und
das Erfassen des Mikroorganismus in der Probemischung.

10. Verfahren nach Anspruch 9, wobei das Erfassen Folgendes umfasst: das Erhalten gesamter DNA aus dem Mikroorganismus; das Kontaktieren der gesamten DNA mit einer Testreplikationszusammensetzung unter Bildung einer ersten Reaktionsmischung und mit einer positiven Kontrollreplikationsmischung unter Bildung einer zweiten Reaktionsmischung; das Thermozyklieren der ersten Reaktionsmischung und zweiten Reaktionsmischung, wodurch ein DNA-Amplifikationsprodukt hergestellt wird; und das Erfassen des Amplifikationsprodukts, wobei die Testreplikationszusammensetzung eine Polymerase, ein Primerpaar und ein Reagens umfassen kann; die positive Kontrollreplikationszusammensetzung eine Polymerase, mindestens ein Kontrollnucleinsäurefragment, einen einzelnen Primer, der dazu fähig ist, an einen Teil des Kontrollnucleinsäurefragments zu hybridisieren, und das Reagens umfasst; das Reagens erforderlich ist, um die DNA-Amplifikation zu bewirken; und der Mikroorganismus die Gattung Campylobacter, Listeria, Escherichia, Staphylococcus oder Clostiridium umfasst.

11. Verfahren nach Anspruch 9 oder 10, wobei die komplexe Probemischung eine nichtselektiv angereicherte Nahrungsmittelmatrix umfasst; die Testreplikationszusammensetzung oder positive Kontrollreplikationszusammensetzung bevorzugt in einer Tablettenform vorliegt oder ein Interkalationsmittel umfasst; das Vorliegen der Amplifikationsprodukte durch ein Fluoreszenzmittel erfasst wird; und das Interkalationsmittel bevorzugt ein asymmetrischer Cyaninfarbstoff, einschließlich Chinolinium, 1,1'-[1,3-Propandiylbis[(dimethyliminio)-3,1-propandiyl]]bis[4-[(3-methyl-1-2(3H)-benzothiazolyliden)methyl]]-,tetraiodid oder Chinolinium, 4-[(3-Methyl-2(3H)-benzoxazolyliden)methyl]-1-[3-(trimethylammonio)propyl]-diiodid ist.

12. Verfahren nach Anspruch 10 oder 11, wobei der einzelne Primer gleich wie der erste Primer oder der zweite Primer ist; und die Anzahl der Kontollnucleinsäurefragmente 1 bis 10 beträgt.

## Revendications

1. Sac comprenant:
(a) une première feuille de film polymère;
(b) une deuxième feuille de film polymère; dans lequel ladite deuxième feuille est superposée sur ladite première feuille; et dans lequel ladite première feuille et ladite deuxième feuille sont scellées l'une à l'autre directement ou indirectement par un film polymère intermédiaire, définissant ainsi un périmètre scellé formant un sac;
(c) un lieu de rétention à l'intérieur du sac, ledit lieu comprenant un concentré nutritif; et
(d) éventuellement un ou plusieurs lieux de rétention supplémentaires à l'intérieur du sac, chacun des lieux supplémentaires comprenant un additif choisi dans le groupe constitué d'un composé indicateur, d'un colorant, d'un extincteur de luminescence, d'un fixateur, d'un réactif pour une extraction ou une détection de microorganismes et des combinaisons de deux ou plus de ceux-ci; dans lequel le réactif pour une extraction ou une détection de microorganismes est pur ou comprend un ou plusieurs autres additifs, diluants, phages, composants dérivés de phages, anticorps, poly-histamines, domaines de liaison de maltose, peptides d'affinité, aptomères, biotines, streptoavidines ou autres molécules d'affinité;
dans lequel ledit lieu de rétention ou, lorsqu'il est présent, le au moins un desdits lieux de rétention supplémentaires comprend (1) au moins une fermeture cassable interne au périmètre, divisant ledit sac en compartiments séparés ou (2) un sachet; dans lequel ladite fermeture cassable ou ledit sachet est lui-même au moins partiellement composé d'un matériau polymère réactif avec l'eau; et dans lequel ledit matériau polymère réactif avec l'eau se dissout, se rompt, se disperse et/ou se désintègre lors d'un contact avec l'eau.

2. Sac selon la revendication 1, où le sac inclut une fermeture refermable.

3. Sac selon la revendication 1 ou 2, dans lequel:
le lieu comprend une poudre, un granulé, une pastille, une feuille ou une plaque comprenant une matrice de matériau polymère réactif avec l'eau dans lequel le concentré nutritif est mélangé.

4. Sac selon la revendication 3, dans lequel le milieu nutritif est transformé en une pastille, une feuille ou une plaque avant l'application de l'enrobage de matériau polymère réactif avec l'eau.

5. Sac selon la revendication 1, 2, 3 ou 4, dans lequel le lieu ou un des lieux supplémentaires comprend un comprimé ou une capsule capable d'être pulvérisé et/ou dissous pendant un procédé d'homogénéisation.

6. Sac selon la revendication 1, 2, 3, 4 ou 5, comprenant les un ou plusieurs lieux de rétention supplémentaires dans lequel les un ou plusieurs lieux supplémentaires comprennent un antibiotique et le réactif est sous la forme d'un liquide, d'un gel, d'une pâte, d'une poudre sèche, d'un granulé ou d'une autre forme à écoulement libre.

7. Sac selon la revendication 6, dans lequel le réactif est immobilisé sur un support solide comprenant des particules magnétiques ou des particules paramagnétiques.

8. Sac selon la revendication 1, 2, 3, 4, 5, 6 ou 7, comprenant (1) un moyen de guidage pour faciliter l'ouverture du sac pour ajouter un échantillon de culture dans lequel le moyen de guidage comprend au moins une entaille, une perforation ou une combinaison de celles-ci incorporée dans le sac près de la fermeture supérieure ou (2) une base à soufflets permettant au sac de se tenir droit lorsqu'il est rempli.

9. Procédé comprenant:
l'insertion d'un échantillon contenant un microorganisme dans un sac tel que caractérisé dans la revendication 1, 2, 3, 4, 5, 6, 7 ou 8;
la libération d'un concentré nutritif à partir du lieu pour produire un milieu de culture;
l'incubation de l'échantillon dans le milieu de culture pour former un mélange d'échantillon complexe enrichi; et
la détection du microorganisme dans le mélange d'échantillon.

10. Procédé selon la revendication 9, dans lequel la détection comprend l'obtention d'un ADN total à partir du microorganisme; la mise en contact de l'ADN total avec une composition de réplication d'essai pour former un premier mélange de réaction et avec une composition de réplication de témoin positif pour former un deuxième mélange de réaction; le thermocyclage du premier mélange de réaction et du deuxième mélange de réaction produisant ainsi un produit d'amplification d'ADN; et la détection du produit d'amplification dans lequel la composition de réplication d'essai peut comprendre une polymérase, une paire d'amorces et un réactif; la composition de réplication de témoin positif comprend une polymérase, au moins un fragment d'acide nucléique témoin, une amorce unique capable de s'hybrider à une portion du fragment d'acide nucléique témoin et le réactif; le réactif est nécessaire pour effectuer une amplification d'ADN; et le microorganisme inclut le genre de *Campylobacter,* de *Listeria,* d'*Escherichia,* de *Staphylococcus* ou de *Clostridium.*

11. Procédé selon la revendication 9 ou 10, dans lequel le mélange d'échantillon complexe comprend une matrice alimentaire enrichie non sélectivement; la composition de réplication d'essai ou la composition de réplication de témoin positif est de préférence sous une forme de comprimé ou comprend un agent intercalant; la présence des produits d'amplification est détectée par un moyen fluorescent; et l'agent intercalant est de préférence un colorant de cyanine asymétrique incluant le Quinolinium, 1,1'-[1,3-propanediylbis-[(diméthyliminio)-3,1-propanediyl]]bis[4-[(3-méthyl-1-2(3H)-benzothiazolylidène)méthyl]]-tétraiodure ou le Quinolinium, 4-[(3-méthyl-2(3H)-benzoxazolylidène)méthyl]-1-[3-(triméthylammonio)propyl]-diiodure.

12. Procédé selon la revendication 10 ou 11, dans lequel l'amorce unique est la même que la première amorce ou la deuxième amorce; et le nombre des fragments d'acide nucléique témoin est de 1 à 10.
